# EUROPEAN PATENT APPLICATION

(11) **EP 4 685 447 A1**
(43) Date of publication of application: **28.01.2026**
(21) Application number: 25187413.7
(22) Date of filing: 03.07.2025
(51) Int. Cl.: G01L 19/00, A61M 5/142, A61M 5/168, G01L 19/14

(54) **SENSOR WITH ECCENTRIC SENSING COLUMN**

(30) Priority: 22.07.2024 IN 202411055694
(71) Applicant: Honeywell International Inc., Charlotte, NC 28202 (US)
(72) Inventor: S, Vijayakumar, Charlotte, 28202 (US); JAIN, Rajani Niranjan, Charlotte, 28202 (US); MURALI, Hariprasad Padubidri, Charlotte, 28202 (US)
(74) Representative: Haseltine Lake Kempner LLP

(57) **Abstract**

Example embodiments described herein may include a housing for at least one sensing element defining an inclined and/or eccentric sensing column cavity. The sensing column cavity may be filled with a gel material for imparting force and/or pressure to a sensing element. In some embodiments, the sensing column cavity may have a first width at the end of the sensing column cavity proximate to a PCB. The sensing column cavity may have a second width at the end of the sensing column cavity proximate to a protective cap. The first width may be smaller than the second width, wherein the first width defines a diameter of a substantially circular profile of the sensing column cavity and the second width defines a major axis of a substantially elliptical profile of the sensing column cavity. The sensing column cavity may comprise one or more additional widths defining one or more additional profiles.

## Description

### FIELD OF THE INVENTION

Embodiments of the present disclosure generally relate to devices comprising eccentric and/or inclined sensing columns.

### BACKGROUND

There are many different devices for measuring pressure and/or force, for example, of fluids being transported by fluid flow tubes. Infusion pumps of hemodialysis machines, for example, comprise pressure and/or force sensors to measure properties of fluids being transported by fluid flow tubes (e.g., such as blood, dialysate, medication, etc.). Applicant has identified many technical challenges and difficulties associated with such devices comprising eccentric and/or inclined sensing columns for measuring pressure and/or force. Through applied effort, ingenuity, and innovation, many of these identified problems have been solved by developing solutions that are included in embodiments of the present disclosure, many examples of which are described in detail herein.

### BRIEF SUMMARY

Various example embodiments described herein relate to devices comprising eccentric and/or inclined sensing columns.

In accordance with various embodiments of the present disclosure, an apparatus is provided. In some embodiments, the apparatus comprises a housing defining an inclined and eccentric sensing column cavity comprising a gel material, the housing further comprising: a printed circuit board (PCB); at least one sensing element wire-bonded to the PCB; at least one application specific integrated circuit (ASIC) wire-bonded to the PCB; and a protective cap mechanically coupled to the housing via one or more protruding edges of the housing and one or more corresponding edges of the protective cap.

In some embodiments, the sensing column cavity has a first width at a side of the sensing column cavity proximate to the PCB and a second width at a side of the sensing column cavity proximate to the protective cap, and wherein the first width is smaller than the second width.

In some embodiments, the first width defines a diameter of a circular profile of the sensing column cavity.

In some embodiments, the second width defines a major axis of an elliptical profile of the sensing column cavity.

In some embodiments, the sensing column cavity further comprises one or more widths defining one or more profiles.

In some embodiments, the PCB further comprises protruding standoff features configured to mechanically couple to the housing such that the housing is substantially perpendicular to the PCB.

In some embodiments, the sensing column cavity is inclined in two or more directions.

In accordance with various embodiments of the present disclosure, a system is provided. In some embodiments, the system comprises: a sensor device, the sensor device comprising: a housing defining an inclined and eccentric sensing column cavity comprising a gel material, the housing further comprising: a printed circuit board (PCB); at least one sensing element wire-bonded to the PCB; at least one application specific integrated circuit (ASIC) wire-bonded to the PCB; and a protective cap mechanically coupled to the housing via one or more protruding edges of the housing and one or more corresponding edges of the protective cap; and at least one infusion pump, wherein the at least one infusion pump comprises at least one fluid flow tube containing a fluid to be measured by the sensor device.

In some embodiments, the sensing column cavity has a first width at a side of the sensing column cavity proximate to the PCB and a second width at a side of the sensing column cavity proximate to the protective cap, and wherein the first width is smaller than the second width.

In some embodiments, the first width defines a diameter of a circular profile of the sensing column cavity.

In some embodiments, the second width defines a major axis of an elliptical profile of the sensing column cavity.

In some embodiments, the sensing column cavity further comprises one or more widths defining one or more profiles.

In some embodiments, the PCB further comprises protruding standoff features configured to mechanically couple to the housing such that the housing is substantially perpendicular to the PCB.

In some embodiments, the sensing column cavity is inclined in two or more directions.

In accordance with various embodiments of the present disclosure, a method is provided. In some embodiments, the method comprises: coupling, via one or more adhesives, a housing for at least one sensing element to a printed circuit board (PCB), the housing defining an inclined and eccentric sensing column cavity comprising a gel material, the housing further comprising: the at least one sensing element wire-bonded to the PCB; and at least one application specific integrated circuit (ASIC) wire-bonded to the PCB; and coupling, via one or more protruding edges of the housing and one or more corresponding edges of a protective cap, the housing to the protective cap.

In some embodiments, the sensing column cavity has a first width at a side of the sensing column cavity proximate to the PCB and a second width at a side of the sensing column cavity proximate to the protective cap, and wherein the first width is smaller than the second width.

In some embodiments, the first width defines a diameter of a particular circular profile of the sensing column cavity.

In some embodiments, the second width defines a major axis of an elliptical profile of the sensing column cavity.

In some embodiments, the sensing column cavity further comprises one or more widths defining one or more profiles.

In some embodiments, the method further comprises coupling, via protruding standoff features comprised by the PCB, the housing to the PCB such that the housing is substantially perpendicular to the housing.

### BRIEF DESCRIPTION OF THE DRAWINGS

The description of the illustrative embodiments may be read in conjunction with the accompanying figures. It will be appreciated that, for simplicity and clarity of illustration, elements illustrated in the figures have not necessarily been drawn to scale, unless described otherwise. For example, the dimensions of some of the elements may be exaggerated relative to other elements, unless described otherwise. Embodiments incorporating teachings of the present disclosure are shown and described with respect to the figures presented herein, in which:
FIG. 1 is a cross-sectional view of an example sensor device having an eccentric, inclined sensing column;
FIG. 2 is a perspective view of an example covered sensor device having an eccentric, inclined sensing column;
FIG. 3A is a top-down view of an example covered sensor device having an eccentric, inclined sensing column;
FIG. 3B is a top-down view of an example uncovered sensor device having an eccentric, inclined sensing column; and
FIG. 4 is a flowchart of an example method for constructing a sensor device having an eccentric, inclined sensing column, in accordance with some embodiments of the present disclosure.

### DETAILED DESCRIPTION OF THE INVENTION

Some embodiments of the present disclosure will now be described more fully hereinafter with reference to the accompanying drawings, in which some, but not all embodiments of the disclosure are shown. Indeed, these disclosures may be embodied in many different forms and should not be construed as limited to the embodiments set forth herein; rather, these embodiments are provided so that this disclosure will satisfy applicable legal requirements. Like numbers refer to like elements throughout.

As used herein, terms such as "front," "rear," "top," "bottom," "left," "right," etc. are used for explanatory purposes in the examples provided below to describe the relative position of certain components or portions of components. Furthermore, as would be evident to one of ordinary skill in the art in light of the present disclosure, the terms "substantially" and "approximately" indicate that the referenced element or associated description is accurate to within applicable engineering tolerances.

As used herein, the term "comprising" means including but not limited to and should be interpreted in the manner it is typically used in the patent context. Use of broader terms such as comprises, includes, and having should be understood to provide support for narrower terms such as consisting of, consisting essentially of, and comprised substantially of.

The phrases "in one embodiment," "according to one embodiment," "in some embodiments," and the like generally mean that the particular feature, structure, or characteristic following the phrase may be included in at least one embodiment of the present disclosure and may be included in more than one embodiment of the present disclosure (importantly, such phrases do not necessarily refer to the same embodiment).

The phrases "in one example," "according to one example," "in some examples," and the like generally mean that the particular feature, structure, or characteristic following the phrase may be included in at least one example of the present disclosure and may be included in more than one example of the present disclosure (importantly, such phrases do not necessarily refer to the same example).

If the specification states a component or feature "may," "can," "could," "should," "would," "preferably," "possibly," "typically," "optionally," "for example," "as an example," "in some examples," "often," or "might" (or other such language) be included or have a characteristic, that specific component or feature is not required to be included or to have the characteristic. Such component or feature may be optionally included in some examples, or it may be excluded.

The word "example" or "exemplary" is used herein to mean "serving as an example, instance, or illustration." Any implementation described herein as "example" or "exemplary" is not necessarily to be construed as preferred or advantageous over other implementations.

The term "electrically coupled," "electrically coupling," "electrically couple," "electrically connected," "electrically connecting," "electrically connect," "in communication with," or "in electronic communication with" in the present disclosure refers to two or more elements or components being connected through wired means and/or wireless means, such that signals, electrical voltage/current, data and/or information may be transmitted to and/or received from these elements or components.

The term "in fluid communication with" in the present disclosure refers to two or more elements or components being connected through one or more paths or pathways, such that a fluid or other flowing media may be input to and/or output from these elements or components.

The term "component" may refer to an article, a device, or an apparatus that may comprise one or more surfaces, portions, layers and/or elements. For example, an example component may comprise one or more substrates that may provide underlying layer(s) for the component and may comprise one or more elements that may form part of and/or are disposed on top of the substrate. In the present disclosure, the term "element" may refer to an article, a device, or an apparatus that may provide one or more functionalities.

The term "sensor" refers to a component that may detect, measure, and/or identify any one or more attributes or characteristics of an environment or media, including but not limited to pressure(s) and/or force(s).

In some examples, pressure and/or force sensors are limited by various features. For example, a pressure and/or force sensor may comprise a vertical (e.g., perpendicular to a substrate) sensing column, wherein the column may be filled with a gel material. The pressure and/or force sensor may have a circular sensing surface which may be in contact (e.g., either via direct contact or through a protective cap proximate to the sensing surface) with a fluid flow tube containing materials to be measured. However, such configurations of pressure and/or force sensors, in some examples, have large footprints resulting in larger devices.

Embodiments of the present disclosure, in some examples, provide devices comprising eccentric and/or inclined sensing columns.

Example embodiments of the devices described herein may include a housing for at least one sensing element. The housing may define an inclined and eccentric sensing column cavity. The sensing column cavity may be filled with a gel material for imparting force and/or pressure to a sensing element (e.g., a sense die) and/or for protecting electronics comprised within the devices described herein. The example embodiments of the devices described herein may further comprise a printed circuit board (PCB) and/or a protective cap. The housing may further comprise at least one sensing element wire-bonded to the PCB. The housing may further comprise at least one application specific integrated circuit (ASIC) wire-bonded to the PCB. The protective cap may be mechanically coupled to the housing via one or more protruding edges of the housing and/or one or more corresponding edges of the protective cap.

In the example embodiments of the devices described herein, the sensing column cavity may have a first width at the end of the sensing column cavity proximate to the PCB. The sensing column cavity may have a second width at the end of the sensing column cavity proximate to the protective cap. The first width may be smaller than the second width. The first width may define a diameter of a substantially circular profile of the sensing column cavity. The second width may define a major axis of a substantially elliptical profile of the sensing column cavity. The sensing column cavity may further comprise one or more additional widths defining one or more additional profiles (e.g., between the substantially circular profile and the substantially elliptical profile).

In the example embodiments of the devices described herein, the PCB may further comprise protruding standoff features configured to mechanically couple to the housing, such that the housing is substantially perpendicular to the PCB.

In the example embodiments of the devices described herein, the sensing column cavity may be inclined in two or more directions.

Example embodiments of the systems described herein may include a sensor device, such as the devices described herein, and at least one infusion pump. For example, the at least one infusion pump may include at least one fluid flow tube containing a fluid to be measured by the sensor device.

Example embodiments of the methods described herein may include a method for constructing a sensor device, such as the devices described herein.

Example embodiments of the present disclosure, in some examples, provide for a fluid flow tube being in direct mechanical contact with a protective cap of a sensor device. The protective cap of the sensor device may impart at least a portion of any received pressure and/or force to a gel material contained within a sensing column cavity of the sensor device, and the gel material may impart at least a portion of any received pressure and/or force to a sensing element comprised by the sensor device.

Example embodiments of the present disclosure provide for monitoring and/or regulating dialysate flow externally to a dialysis filter, such that the dialysis filter may capture contaminants within a patient's blood. Example embodiments of the present disclosure provide for improving treatment efficiency and/or reducing time used to remove fluid from a peritoneum. Example embodiments of the present disclosure provide for monitoring deliver of fluids, medications, and/or nutrients to a patient. Example embodiments of the present disclosure provide for detecting blockages within a fluid flow tube and/or determining that a bag containing fluids and/or nutrients can be changed.

To address challenges and limitation associated with devices for measuring pressure and/or force, various examples of the present disclosure may be provided. For examples, various examples of the present disclosure may provide example devices and/or systems for measuring pressure and/or force, and associated methods. In some embodiments, the disclosure may provide a device for measuring pressure and/or force having an eccentric and/or inclined sensing column.

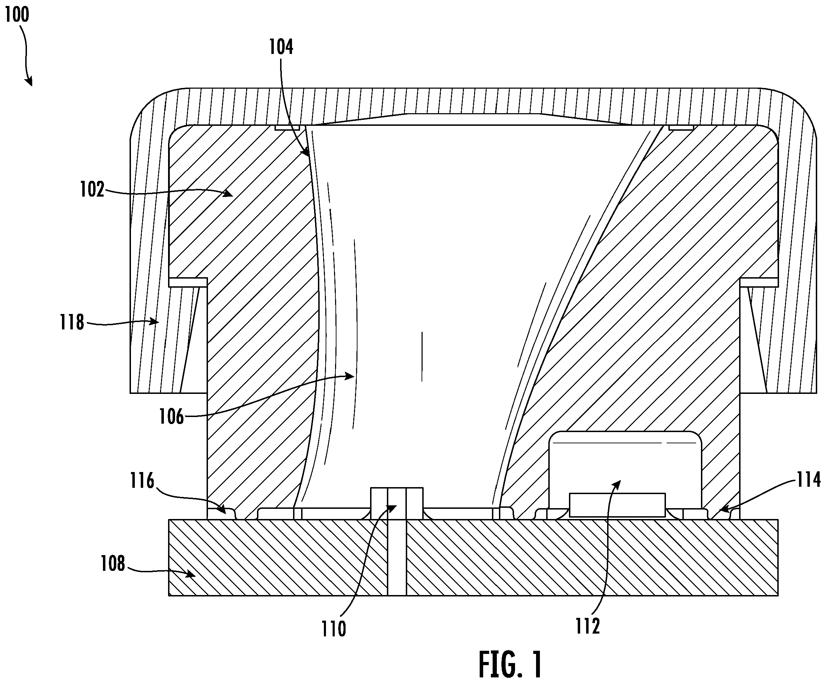

Referring now to FIG. 1, a cross-sectional view of an example sensor device 100 having an eccentric, inclined sensing column is provided. The sensor device 100 may have an eccentric sensing column, wherein at least one end (e.g., surface, profile, and/or the like) of the sensing column near a medium being measured (e.g., either in direct contact with a fluid flow tube containing the medium being measured or separated by a protective cap covering at least a portion of the sensor device 100) may be eccentric and/or substantially elliptical. At least one other end (e.g., surface, profile, and/or the like) of the sensing column proximate to a substrate (e.g., a printed circuit board (PCB)) may be substantially circular. The sensor device 100 may have an inclined sensing column, wherein the sensing column may be angled with respect to a substrate. For example, the angle may range from 0 degrees to 180 degrees (e.g., 0 degrees or more, 180 degrees or less, more preferably ranging from 0 degrees to 90 degrees, preferably approximately 60 degrees). The sensing column may be inclined in one or more directions. The sensor device 100 may have an eccentric sensing column, an inclined sensing column, or an eccentric and inclined sensing column.

The sensor device 100 may comprise a housing 102, a sensing column 104, a gel material 106, a substrate 108, a sensing element 110, an application specific integrated circuit (ASIC) 112, at least one standoff feature 114, at least one amount of adhesive 116, and a protective cap 118. Although the example of FIG. 1 shows one housing 102, one sensing column 104, one amount of gel material 106, one substrate 108, one sensing element 110, one ASIC 112, and one protective cap 118, any number of these elements may be present in the sensor device 100.

The housing 102 may be comprised of plastic, metal (e.g., stainless steel), and/or other materials. The housing 102 may define a sensing column cavity, wherein the sensing column cavity has two or more profiles (e.g., surfaces, cross-sections, and/or the like): (a) a first profile on the end of the sensing column cavity proximate to the substrate 108, (b) a second profile on the end of the sensing column cavity proximate to the protective cap 118, and/or (c) one or more additional profiles disposed between the first profile and the second profile. The housing 102 may further define a cavity for the ASIC 112 corresponding to the dimensions of the ASIC 112. The housing 102 may couple to the protective cap 118 via at least one protruding edge of the housing 102.

The sensing column 104 may be comprised of a sensing column cavity defined by the housing 102. The sensing column 104 may be further comprised of the gel material 106. Together, the sensing column cavity and the gel material 106 comprise the sensing column 104. The sensing column 104 may be eccentric (e.g., substantially elliptical, non-circular, and/or the like) on one end. The sensing column 104 may be inclined (e.g., angled with respect to the substrate 108). For example, the angle may range from 0 degrees to 180 degrees (e.g., 0 degrees or more, 180 degrees or less, more preferably ranging from 0 degrees to 90 degrees, preferably approximately 60 degrees). In the example of FIG. 1, the sensing column 104 is both eccentric and inclined, although the sensing column 104 may be either eccentric or inclined. The sides of the sensing column 104 may be curved (e.g., curved inward toward the center of the sensing column 104). The sides of the sensing column 104 may be substantially straight (not shown in FIG. 1). One end of the sensing column 104 (e.g., its "top" end) may be in direct contact with the protective cap 118, such that the gel material 106 may receive forces and/or pressures imparted onto the protective cap 118. The other end of the sensing column 104 (e.g., its "bottom" end) may be in direct contact with the substrate 108, such that the gel material 106 may cover the sensing element 110 and its wired connections to the substrate 108.

The gel material 106 may be configured to receive force and/or pressure of a medium being measured (e.g., a fluid being transported by a fluid flow tube) through the protective cap 118. The gel material 106 may transfer such force and/or pressure to the sensing element 110, such that the sensing element 110 may measure the force and/or pressure. The gel material 106 may further protect the wired connections between the sensing element 110 and the substrate 108. The gel material 106 may be dispensed on one side of the sensing column 104 while letting air escape from the other side to reduce bubbles trapped getting trapped in the gel material 106.

The substrate 108 may be a printed circuit board (PCB). The substrate 108 may be comprised of flame retardant woven glass-reinforced epoxy resin (FR4), thick film network (TFN), and/or other materials. The substrate 108 may comprise at least one standoff feature 114. The at least one standoff feature 114 may comprise two or more standoff features. The two or more standoff features may be distributed over the surface area of the regions of the housing 102 which come into direct physical contact with the substrate 108. The at least one standoff feature 114 may be at least one protruding portion of the housing 102 which aligns with at least one corresponding region of the substrate 108. In some examples, the housing 102 may be coupled to the substrate 108 via the at least one amount of adhesive 116, the thickness of which may cause the housing 102 to become tilted (e.g., not substantially perpendicular to the substrate 108). The at least one standoff feature 114 may be configured to mechanically couple to (e.g., snap into) the corresponding region of the substrate 108. The at least one standoff feature 114 may be configured to cause the housing 102 and the substrate 108 to be substantially perpendicular to one another (e.g., such that they have an angle from 60 - 120 degrees between them, more preferably 80 - 100 degrees, preferably 90 degrees).

The sensing element 110 may be a force sensing element and/or a pressure sensing element. The sensing element 110 may be a piezoresistive sensing element. The sensing element 110 may be a sense die. The sensing element 110 may be configured to determine the force and/or pressure imparted by the gel material 106. The sensing element 110 may be wire-bonded to the substrate 108. The electrical connections between the sensing element 110 and the substrate 108 may be encapsulated by the gel material 106. The electrical connections between the sensing element 110 and the substrate 108 may be protected by the gel material 106. An end of the sensing column 104 (e.g., the "bottom" end) may be in direct contact with the substrate 108 such that the substantially circular profile of the end of the sensing column 104 is substantially centered on the sensing element 110.

The ASIC 112 may be wire-bonded to the substrate 108. The housing 102 may define a cavity corresponding to the dimensions of the ASIC 112. The ASIC 112 may mechanically couple to the housing via the cavity corresponding to the dimensions of the ASIC 112.

The at least one amount of adhesive 116 may comprise two or more dispensed amounts of adhesive, for example, adhesive may be dispensed on two or more locations for coupling the housing 102 and the substrate 108. The at least one amount of adhesive 116 may cause the housing 102 and the substrate 108 to be tilted relative to one another. The at least one standoff feature 114 may be configured to counteract any such tilting, causing the housing 102 and the substrate 108 to be substantially perpendicular to one another.

The protective cap 118 may be configured to cover the housing 102 and the gel material 106 to protect the housing 102 and/or the gel material 106 from the environment. The protective cap 118 may mechanically couple to the housing 102 via at least one protruding edge of the housing 102 which may snap into the protective cap 118. The protective cap 118 may be further configured to receive a force and/or pressure imparted by a medium being measured (e.g., a fluid being transported by a fluid flow tube) and impart that force and/or pressure to the gel material 106, which, in turn, imparts the force and/or pressure to the sensing element 110.

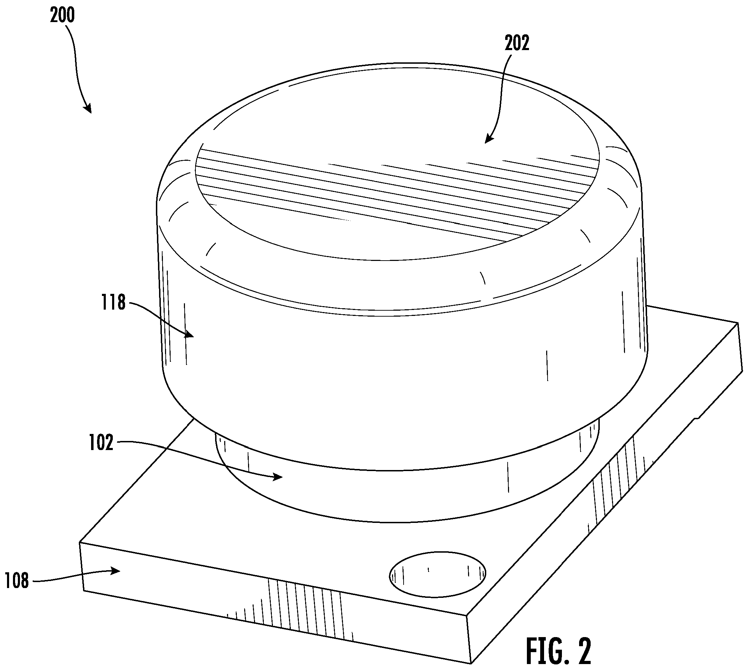

Referring now to FIG. 2, a perspective view 200 of an example covered sensor device having an eccentric, inclined sensing column is provided. The perspective view 200 shows the substrate 108, the housing 102, the protective cap 118, and a surface 202 of the protective cap 118. The surface 202 of the protective cap 118 may be configured to be in direct physical contact with the fluid flow tube transporting the medium being measured. The surface 202 may be configured to receive a force and/or pressure imparted by the medium being measured and impart that force and/or pressure to the gel material 106. The gel material 106 may be configured to impart the received force and/or pressure to the sensing element 110, which may be configured to measure the force and/or pressure.

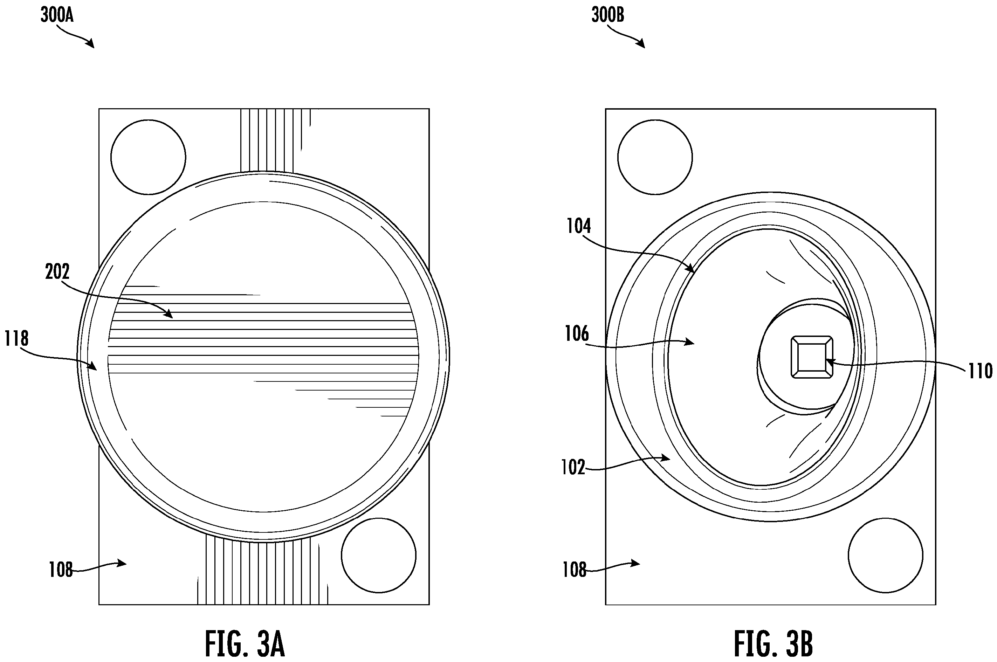

FIGS. 3A-3B provide top-down views of a covered and uncovered sensor device, respectively.

Referring now to FIG. 3A, a top-down view 300A of an example covered sensor device having an eccentric, inclined sensing column is provided. The top-down view 300A shows the substrate 108, the protective cap 118, and the surface 202 of the protective cap 118. The sensor device may be substantially circular. The protective cap 118 and the surface 202 may be substantially circular. The substrate 108 may be configured to define one or more substantially circular holes for coupling the sensor device to one or more other locations (e.g., devices). The one or more substantially circular holes may comprise two or more substantially circular holes.

Referring now to FIG. 3B, a top-down view 300B of an example uncovered sensor device having an eccentric, inclined sensing column is provided. The top-down view 300B shows the substrate 108, the housing 102, the sensing column 104, the gel material 106, and the sensing element 110. As described with respect to FIG. 3A, the substrate 108 may define one or more substantially circular holes for coupling the sensor device to one or more other locations (e.g., devices). As described with respect to FIG. 1, the housing 102 may define a sensing column cavity. The sensing column 104 may comprise two ends: (a) a first end (e.g., a "bottom" end) proximate to the substrate 108, and (b) a second end (e.g., a "top" end) proximate to the protective cap 118. The first end of the sensing column 104 may comprise a first profile, wherein the first profile is substantially circular. The first end of the sensing column 104 may be centered around the sensing element 110. The second end of the sensing column 104 may comprise a second profile, wherein the second profile is eccentric and/or substantially elliptical. The second end of the sensing column 104 may be configured to not be centered on the sensing element 104. The sensing column 104 may be angled (e.g., with respect to the substrate 108). The sensing column 104 being angled and having a substantially elliptical top surface may allow for a more compact design of the sensor device, as more components (e.g., the ASIC 112) may be able to fit on the substrate 108 in areas that would have otherwise been filled with a completely centered and/or substantially perpendicular sensing column.

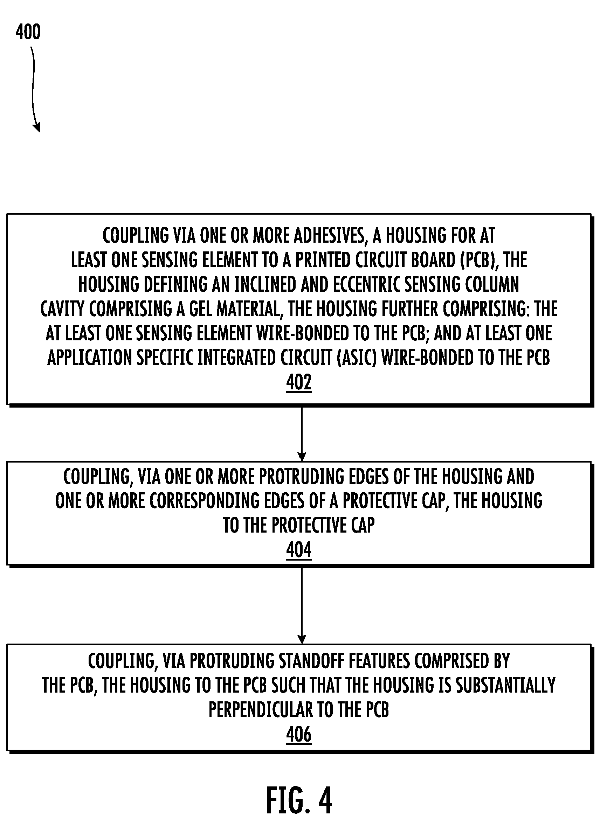

Referring now to FIG. 4, a flowchart of an example method for constructing a sensor device having an eccentric, inclined sensing column is provided.

At step/operation 402, a housing (e.g., the housing 102) for at least one sensing element (e.g., the sensing element 110) may be coupled, via one or more adhesives (e.g., the at least one amount of adhesive 116), to a PCB (e.g., the substrate 108). The housing may define an inclined and/or eccentric sensing column cavity (e.g., the sensing column cavity of the sensing column 104) comprising a gel material (e.g., the gel material 106). The housing may further comprise the at least one sensing element wire-bonded to the PCB. The housing may further comprise at least one application specific integrated circuit (ASIC) (e.g., the ASIC 112) wire-bonded to the PCB.

At step/operation 404, the housing may be coupled to a protective cap (e.g., the protective cap 118) via one or more protruding edges of the housing and one or more corresponding edges of the protective cap.

At step/operation 406, the housing may be coupled to the PCB via protruding standoff features (e.g., the at least one standoff feature 114) comprised by the PCB such that the housing is substantially perpendicular to the PCB.

Operations and processes described herein support combinations of means for performing the specified functions and combinations of operations for performing the specified functions. It will be understood that one or more operations, and combinations of operations, may be implemented by special purpose hardware-based computer systems which perform the specified functions, or combinations of special purpose hardware and computer instructions.

In some example embodiments, certain ones of the operations herein may be modified or further amplified as described below. Moreover, in some embodiments additional optional operations may also be included. It should be appreciated that each of the modifications, optional additions or amplifications described herein may be included with the operations herein either alone or in combination with any others among the features described herein.

The foregoing method and process descriptions are provided merely as illustrative examples and are not intended to require or imply that the steps of the various embodiments must be performed in the order presented. As will be appreciated by one of skill in the art the order of steps in the foregoing embodiments may be performed in any order. Words such as "thereafter," "then," "next," and similar words are not intended to limit the order of the steps; these words are simply used to guide the reader through the description of the methods. Further, any reference to claim elements in the singular, for example, using the articles "a," "an" or "the," is not to be construed as limiting the element to the singular and may, in some instances, be construed in the plural.

While various embodiments in accordance with the principles disclosed herein have been shown and described above, modifications thereof may be made by one skilled in the art without departing from the teachings of the disclosure. The embodiments described herein are representative only and are not intended to be limiting. Many variations, combinations, and modifications are possible and are within the scope of the disclosure. Alternative embodiments that result from combining, integrating, and/or omitting features of the embodiment(s) are also within the scope of the disclosure. Accordingly, the scope of protection is not limited by the description set out above, but is defined by the claims which follow, that scope including all equivalents of the subject matter of the claims. Each and every claim is incorporated as further disclosure into the specification and the claims are embodiment(s) of the present disclosure. Furthermore, any advantages and features described above may relate to specific embodiments but shall not limit the application of such issued claims to processes and structures accomplishing any or all of the above advantages or having any or all of the above features.

In addition, the section headings used herein are provided for consistency with the suggestions under 37 C.F.R. § 1.77 or to otherwise provide organizational cues. These headings shall not limit or characterize the disclosure set out in any claims that may issue from this disclosure. For instance, a description of a technology in the "Background" is not to be construed as an admission that certain technology is prior art to any disclosure in this disclosure. Neither is the "Summary" to be considered as a limiting characterization of the disclosure set forth in issued claims. Furthermore, any reference in this disclosure to "disclosure" or "embodiment" in the singular should not be used to argue that there is only a single point of novelty in this disclosure. Multiple embodiments of the present disclosure may be set forth according to the limitations of the multiple claims issuing from this disclosure, and such claims accordingly define the disclosure, and their equivalents, which are protected thereby. In all instances, the scope of the claims shall be considered on their own merits in light of this disclosure but should not be constrained by the headings set forth herein.

Also, systems, subsystems, apparatuses, techniques, and methods described and illustrated in the various embodiments as discrete or separate may be combined or integrated with other systems, modules, techniques, or methods without departing from the scope of the present disclosure. Other devices or components shown or discussed as coupled to, or in communication with, each other may be indirectly coupled through some intermediate device or component, whether electrically, mechanically, or otherwise. Other examples of changes, substitutions, and alterations are ascertainable by one skilled in the art and could be made without departing from the scope disclosed herein.

Many modifications and other embodiments of the disclosure set forth herein will come to mind to one skilled in the art to which these embodiments pertain having the benefit of teachings presented in the foregoing descriptions and the associated figures. Although the figures only show certain components of the apparatuses and systems described herein, various other components may be used in conjunction with the components and structures disclosed herein. Therefore, it is to be understood that the disclosure is not to be limited to the specific embodiments disclosed and that modifications and other embodiments are intended to be included within the scope of the appended claims. For example, the various elements or components may be combined, rearranged, or integrated in another system or certain features may be omitted or not implemented. Moreover, the steps in any method described above may not necessarily occur in the order depicted in the accompanying drawings, and in some cases one or more of the steps depicted may occur substantially simultaneously, or additional steps may be involved. Although specific terms are employed herein, they are used in a generic and descriptive sense only and not for purposes of limitation.

## Claims

1. An apparatus comprising:
a housing defining an inclined and eccentric sensing column cavity comprising a gel material, the housing further comprising:
a printed circuit board (PCB);
at least one sensing element wire-bonded to the PCB;
at least one application specific integrated circuit (ASIC) wire-bonded to the PCB; and
a protective cap mechanically coupled to the housing via one or more protruding edges of the housing and one or more corresponding edges of the protective cap.

2. The apparatus of claim 1, wherein the sensing column cavity has a first width at a side of the sensing column cavity proximate to the PCB and a second width at a side of the sensing column cavity proximate to the protective cap, and wherein the first width is smaller than the second width.

3. The apparatus of any of claims 1or 2, wherein the first width defines a diameter of a circular profile of the sensing column cavity.

4. The apparatus of any of claims 1 to 3, wherein the second width defines a major axis of an elliptical profile of the sensing column cavity.

5. The apparatus of any of claims 1 to 4, wherein the sensing column cavity further comprises one or more widths defining one or more profiles.

6. The apparatus of any of claims 1 to 5, wherein the PCB further comprises protruding standoff features configured to mechanically couple to the housing such that the housing is substantially perpendicular to the PCB.

7. The apparatus of any of claims 1 to 6, wherein the sensing column cavity is inclined in two or more directions.

8. A system comprising:
a sensor device, the sensor device comprising:
a housing defining an inclined and eccentric sensing column cavity comprising a gel material, the housing further comprising:
a printed circuit board (PCB);
at least one sensing element wire-bonded to the PCB;
at least one application specific integrated circuit (ASIC) wire-bonded to the PCB; and
a protective cap mechanically coupled to the housing via one or more protruding edges of the housing and one or more corresponding edges of the protective cap; and
at least one infusion pump, wherein the at least one infusion pump comprises at least one fluid flow tube containing a fluid to be measured by the sensor device.

9. The system of claim 8, wherein the sensing column cavity has a first width at a side of the sensing column cavity proximate to the PCB and a second width at a side of the sensing column cavity proximate to the protective cap, and wherein the first width is smaller than the second width.

10. The system of any of claims 8 or 9, wherein the first width defines a diameter of a circular profile of the sensing column cavity.

11. The system of any of claims 8 to 10, wherein the second width defines a major axis of an elliptical profile of the sensing column cavity.

12. The system of any of claims 8 to 11, wherein the sensing column cavity further comprises one or more widths defining one or more profiles.

13. The system of any of claims 8 to 12, wherein the PCB further comprises protruding standoff features configured to mechanically couple to the housing such that the housing is substantially perpendicular to the PCB.

14. The system of any of claims 8 to 13, wherein the sensing column cavity is inclined in two or more directions.

15. A method comprising:
coupling, via one or more adhesives and one or more standoff features, a housing for at least one sensing element to a printed circuit board (PCB) such that the housing is substantially perpendicular to the PCB, the housing defining an inclined and eccentric sensing column cavity comprising a gel material, and the housing further comprising:
the at least one sensing element wire-bonded to the PCB; and
at least one application specific integrated circuit (ASIC) wire-bonded to the PCB;
coupling, via one or more protruding edges of the housing and one or more corresponding edges of a protective cap, the housing to the protective cap;
wherein the sensing column cavity has a first width at a side of the sensing column cavity proximate to the PCB and a second width at a side of the sensing column cavity proximate to the protective cap, and wherein the first width is smaller than the second width;
wherein the first width defines a diameter of a circular profile of the sensing column cavity and the second width defines a major axis of an elliptical profile of the sensing column cavity; and
wherein the sensing column cavity further comprises one or more widths defining one or more profiles.
